# EUROPEAN PATENT APPLICATION

(11) **EP 1 362 912 A1**
(43) Date of publication of application: **19.11.2003**
(21) Application number: 02712417.1
(22) Date of filing: 18.02.2002
(51) Int. Cl.: C12N 15/09, C12Q 1/68, C12M 1/00

(54) **METHOD OF AMPLIFYING MRNA AND CDNA IN MICROQUANTITIES**

(30) Priority: 19.02.2001 JP 2001041428
(71) Applicant: JAPAN SCIENCE AND TECHNOLOGY CORPORATION, Kawaguchi-shi, Saitama 332-0012 (JP)
(72) Inventor: TAKIGUCHI, Masaki, Funabashi-shi, Chiba 274-0824 (JP)
(74) Representative: Holliday, Louise Caroline
(86) International application number: JP0201360
(87) International publication number: WO02066632

(57) **Abstract**

The present invention provides a method for amplifying mRNA in ultramicroquantity by approximately 10⁸ times which is possible to apply for generating a cDNA library, subtraction cloning, generating and analyzing a microarray, and analyzing a gene expression. After making mRNA of sample adsorbed to oligo (dT)-bound magnetic beads, a double-stranded cDNA is synthesized on magnetic beads, an antisense strand cDNA-bound magnetic beads are eliminated after adding a linker containing T7 promoter sequence at the 5' end, by using a sense strand cDNA in supernatant as a template and using an oligo (dT) primer wherein a linker containing SP6 promoter sequence is added, a double-stranded cDNA is synthesized again, the cDNA mixture is amplified by PCR using a known sequence at a linker part of the both ends of said double-stranded cDNA as a primer. In addition, sense strand/antisense strand cRNA is synthesized by T7 or SP6 polymerase using said cDNA mixture.

SEQUENCE LISTING

<110> JAPAN SCIENCE AND TECHNOLOGY CORPORATION

<120> Amplification method of a very small quantity mRNA

<130> YG13-43PCT

<140>
<141>

<150> JP 2001/041428
<151> 2001-02-19

<160> 8

<170> PatentIn Ver. 2.1

<210> 1
<211> 52
<212> DNA
<213> Artificial Sequence

<220>
<223> Description of Artificial Sequence:MSMAP-5'-T7
      linker upper strand

<400> 1
ccggaatcga ttaatacgac tcactatagg gagatgtcta gaatctcgag tc          52

<210> 2
<211> 50
<212> DNA
<213> Artificial Sequence

<220>
<223> Description of Artificial Sequence:MSMAP-5'-T7
      linker lower strand

<400> 2
gactcgagat tctagacatc tccctatagt gagtcgtatt aatcgattcc          50


<210> 3
<211> 68
<212> DNA
<213> Artificial Sequence

<220>
<223> Description of Artificial Sequence:MSMAP-3'-SP6
      primer

<400> 3
cgataccatg gatttaggtg acactataga ataccggata tcgcggatcc tttttttttt 60
tttttttt                                                          68


<210> 4
<211> 20
<212> DNA
<213> Artificial Sequence

<220>
<223> Description of Artificial Sequence:5' PCR primer

<400> 4
ccggaatcga ttaatacgac                                             20

<210> 5
<211> 20
<212> DNA
<213> Artificial Sequence

<220>
<223> Description of Artificial Sequence:3' PCR primer

<400> 5
cgataccatg gatttaggtg                                                    20

<210> 6
<211> 23
<212> DNA
<213> Artificial Sequence

<220>
<223> Description of Artificial Sequence:T7 promoter
      sequence

<400> 6
taatacgact cactataggg aga                                                23

<210> 7
<211> 23
<212> DNA
<213> Artificial Sequence

<220>
<223> Description of Artificial Sequence:SP6 promoter
      sequence

<400> 7

atttaggtga cactatagaa tac 23

<210> 8
<211> 20
<212> DNA
<213> Artificial Sequence

<220>
<223> Description of Artificial Sequence:T3 promoter
sequence

<400> 8
aattaaccct cactaaaggg 20

## Description

### Technical Field

The present invention relates to a method for amplifying mRNA in microquantity which is present in a sample, or in more detail, a method for sensitively amplifying mRNA in ultramicroquantity expressed in vivo, which is applicable for generation of cDNA library, subtraction cloning, or microarray, and with which a PCR method is combined.

### Background Art

Total number of genomic genes of mammals such as mice and humans has historically been predicted in the approximate range of 100,000, and it is extremely important to comprehensively clone mRNA/cDNA corresponding to said genes not only in practical use but also in basic research from the viewpoint of sequence prediction of a protein encoded by a gene, structure prediction of a gene, and the construction of DNA microarray and the like. In present, therefore, many efforts have been made in worldwide including in Japan and in U.S. For example, in a mouse, around 30,000 types of cDNAs have already been cloned, which are considered to have been mainly derived from mRNAs present in adequate amount in an adult or an embryo, therefore, it is said that the isolation of the remained cDNAs is a future task. In multicellular higher organism, substantial amount of genes is considered to possibly express restrictively in the specialized population of cells. For example, expression of pituitary hormone releasing factor genes in the brain hypothalamus or glucose-regulating hormone genes in a pancreatic islet cells can be seen only in the extremely limited and small number of cells. There is a possibility that a group of genes is expressed only in the restricted region at the specific developmental stage, and there is a possibility that another group of genes is induced only in the cells exposed to a variation of environmental factors such as various types of stresses and infection of pathogen. And then, it is generally pointed out to be useful to diversify the environmental condition for more types of tissues and cells at various developmental stages in order to clone more cDNA species.

It is desirable to isolate a gene, which is industrially useful, in a form of cDNA since it is easy to analyze and prepare its products such as mRNA or a protein. mRNA is used as a starting material for isolation of cDNA, however, it is difficult to prepare cDNA in most cases since most of mRNA species are expressed only in a extremely limited tissue or cell of a living organism as mentioned above, and the amount of mRNA to be obtained is in microquantity. Many methods for amplifying mRNA/cDNA in microquantity have been proposed to date (Dulac, C.& Axel, R. (1995) A novel family of genes encoding putative pheromone receptors in mammals. Cell Vol.83, pp.195-206; Mackler S. A., Brooks, B. P. & Eberwine, J. H. (1992) Stimulus-induced coordinate changes in mRNA abundance in single postsynaptic hippocampal CA1 nuerons. Neuron Vol.9, pp.539-548, etc.), however, most of which are specialized in preparation of a cDNA library or a hybridization probe, and there is no versatility in them.

Besides, as for the methods for promptly and easily detecting a specific nucleic acid sequence in a test sample, the technique related to the following methods are known: methods for producing a double-stranded nucleic acid including a promoter manipulatively binding to the sequence to be detected, and comprising the following steps, (a) oligo nucleotide promoter-primer is obtained, (b) said promoter-primer is contacted with a nucleic acid containing the sequence to be detected under the condition of hybridizing a promoter-primer and such nucleic acid sequence to be detected, (c) an elongation product which is complementary to a nucleic acid sequence to be detected is produced from 3'end of promoter-primer, (d) a product of the step (c) is contacted with a material which has 3' - 5' exonuclease activity, (e) an elongation product, which is complementary to a promoter of promoter-primer, is synthesized from the 3' end of the sequence to be detected (Japanese Laid-Open Patent Application No.11-89600); a method for preserving a polynucleotide immobilized carrier which is useful for various methods for preserving genes including synthesis of sense and antisense mRNA or a single-stranded cDNA, and a gene using such polynucleotide immobilized carrier; a method for producing ss-cDNA, ds-cDNA, sense mRNA or antisense mRNA (WO93/15228). As for the aforementioned method registered in WO93/15228, in spite of the fact that a polynucleotide immobilized carrier is used, a restriction enzyme is used for attachment of an adapter and excision of cDNA from an immobilized carrier, and synthesis efficiency of sense strand cRNA on an immobilized carrier is indefinite. Therefore, a large amount of sense strand cRNA or cDNA cannot be synthesized from mRNA in microquantitiy without a loss by applying said method.

A nervous tissue as a subject of neuroscience is a restricted area in most cases, and an early embryo as a subject of developmental biology has a small number of cells, therefore, mRNA and the like obtained thereof is in extremely microquantity, which makes molecular biological analysis difficult. Currently, a PCR method is most widely applied as a method for amplifying cDNA, however, the number of cycles should be as less as possible since there is a problem in representation of each DNA content. On the other hand, linear amplification by cDNA synthesis is excellent in representation, however, it has a problem in being applied to a sample in extreme microquantity. The object of the present invention is to provide a method for amplifying mRNA in ultramicroquantity expressed in vivo, which has versatility and is applicable for the generation of a cDNA library, subtraction cloning, and microarray.

### Disclosure of the Invention

The present inventors currently attempted to develop a technique for an experiment wherein total RNA in microquantity is amplified, a variety of the specific mRNA is quantified, and at the same time, cDNA library can be easily constructed. After making mRNA in a sample adsorbed to magnetic beads wherein an oligo (dT) is bound, the present inventors synthesized a double-stranded cDNA on said magnetic beads, added a linker having T7 promoter sequence on the 5' end, and then eliminated magnetic beads wherein antisense strand cDNA was bound, synthesized again a double-stranded cDNA by using a sense strand cDNA in supernatant as a template, and using an oligo (dT) primer wherein a linker containing a SP6 promoter sequence was added, amplified a cDNA mixture by conducting PCR with the use of a known sequence in a linker part of both ends of said double-stranded cDNA as a primer, the present inventors subsequently found that mRNA in a sample can be amplified 10⁸-fold by using T7 polymerase and SP6 polymerase, and confirmed that the quantification of mRNA level and the construction of cDNA library is possible by using said method for amplifying mRNA in microquantity. Here, the present invention is completed.

The present invention relates to: a method for amplifying mRNA in microquantity comprising the following processes (1) to (6), (1) a process of making mRNA in a sample adsorbed to a carrier wherein an oligo (dT) is bound, (2) a process of synthesizing an antisense strand cDNA and a sense strand cDNA on a carrier, (3) a process of adding a linker containing the first promoter sequence of the 5' end of at least sense strand among the double-stranded cDNA obtained herein, (4) a process of dissociating said double-stranded cDNA and eliminating an antisense strand cDNA binding to a carrier together with said carrier, (5) a process of synthesizing a double-stranded cDNA by using said sense strand cDNA dissociated herein as a template and using an oligo (dT) primer wherein a linker containing the second promoter sequence is added, (6) a process of amplifying a cDNA mixture by PCR using a sequence of a linker part of the both ends of a double-stranded cDNA as a primer (claim 1); and a method for amplifying mRNA in microquantity comprising the following processes (1) to (7), (1) a process of making mRNA in a sample adsorbed to a carrier wherein an oligo (dT) is bound, (2) a process of synthesizing an antisense strand cDNA and a sense strand cDNA on a carrier, (3) a process of adding a linker containing the first promoter sequence of the 5' end of at least sense strand among the double-stranded cDNA obtained herein, (4) a process of dissociating said double-stranded cDNA and eliminating an antisense strand cDNA binding to a carrier together with said carrier, (5) a process of synthesizing a double-stranded cDNA by using said sense strand cDNA dissociated herein as a template and using an oligo (dT) primer wherein a linker containing the second promoter sequence is added, (6) a process of amplifying a cDNA mixture by PCR using a sequence of a linker part of the both ends of a double-stranded cDNA as a primer, (7) a process of synthesizing a sense strand cRNA and/or an antisense strand cRNA by in vitro transcription system using the first promoter sequence and/or the second promoter sequence of aforementioned (claim 2); a method for amplifying mRNA in microquantity of claim 1 or 2, wherein the carrier is made of magnetic beads (claim 3); a method for amplifying mRNA in microquantity of any of claims 1 to 3, wherein a linker of which the 5' end is a protruding end and the 3' end is a blunt end as a linker containing the first promoter sequence is used (claim 4); a method for amplifying mRNA in microquantity of any of claims 1 to 4, wherein a linker containing a restriction enzyme recognition sequence on the 5' end and/or the 3' end of a promoter sequence as a linker containing the first promoter sequence and/or the second promoter sequence is used (claim 5); a method for amplifying mRNA in microquantity of any of claims 1 to 5, wherein the first promoter sequence is different from the second promoter sequence (claim 6); a method for amplifying mRNA in microquantity of any of claims 1 to 6, of which promoter is the first promoter and/or the second promoter recognized by a RNA polymerase which can specifically transcribe said promoters (claim 7); a method for amplifying mRNA in microquantity of claim 7, wherein a RNA polymerase which can transcribe promoter-specifically is selected among that for T7 promoter, SP6 promoter, or T3 promoter (claim 8); a method for amplifying mRNA in microquantity of any of claims 1 to 8, wherein the linker containing the first promoter sequence comprises the base sequences shown by SEQ ID NO:1 and 2 (claim 9); a method for amplifying mRNA in microquantity of any of claims 1 to 9 wherein an oligo (dT) primer, to which a linker containing the second promoter sequence is added, comprises the base sequence shown by SEQ ID No:3 (claim 10).

The present invention further relates to: a method for cloning a gene by using said method for amplifying mRNA in microquantity of any of claims 1 to 10 (claim 11); a method for subtraction cloning labeling and using at least the one among a sense strand cDNA, an antisense strand cDNA, a sense strand cRNA, or an antisense strand cRNA obtained by the method for amplifying mRNA in microquantity of any of claims 1 to 10 (claim 12); a microarray using at least the one among a sense strand cDNA, an antisense strand cDNA, a sense strand cRNA, or an antisense strand cRNA obtained by the method for amplifying mRNA in microquantity of any of claims 1 to 10 (claim 13); a cDNA library wherein a cDNA obtained by the method for amplifying mRNA in microquantity of any of claims 1 to 10 is introduced into a vector (claim 14); an amplification kit for mRNA in microquantity comprising the followings, a carrier wherein an oligo (dT) is bound, a linker containing the first promoter sequence, and an oligo (dT) primer wherein a linker containing the second promoter sequence different from said first promoter sequence is added (claim 15); an amplification kit for mRNA in microquantity of claim 15, wherein a carrier is made of magnetic beads (claim 16).

### Brief Explanation of the Drawings

Fig. 1 is a drawing showing the outline of each process wherein a cDNA mixture or a cRNA mixture is synthesized by said method for amplifying mRNA in microquantity of the present invention.
Fig. 2 is a drawing showing the structures of a primer or a linker used for said method for amplifying mRNA in microquantity of the present invention.
Fig. 3 is a drawing showing the result of amplifying a cDNA mixture from total RNA by said method for amplifying mRNA in microquantity of the present invention. The second stage of PCR was conducted in 100 µ l of reaction mixture by using 5 µ l of mixture of a PCR product obtained at the first stage. After being reacted for each cycle number, 10 µl was extracted and subjected to agarose gel electrophoresis for analyzing the amplification of a cDNA mixture. A marker for DNA molecular weight was electrophoresed in the lane M.
Fig. 4 is a drawing showing the result of amplifying a cDNA mixture from total RNA serially diluted by said method for amplifying mRNA in microquantity of the present invention. Approximate numbers of the cells corresponding to the amount of total RNA used are also shown. PCR was conducted for 40 cycles only for the first stage. A marker for DNA molecular weight was electrophoresed in the lane M.
Fig. 5 is a drawing showing the result of synthesizing a sense strand or antisense strand cRNA mixture from a cDNA mixture amplified by said method for amplifying mRNA in microquantity of the present invention. Total RNA was electrophoresed as a marker for molecular weight in the lane M.
Fig. 6 is a drawing showing the results of northern hybridization analysis for total RNA and an amplified cDNA mixture. The lanes 1 and 2 show the result of fluorescence staining after electrophoresis of total RNA and its amplified sense strand cRNA mixture derived from a primary culture of rat hepatocytes. After blotting these, arginase mRNA and cRNA were detected (the lanes 3 and 4).
Fig. 7 is a drawing showing the outline of the reverse northern hybridization analysis in the present invention.
Fig. 8 is a drawing showing the result of reverse northern hybridization analysis.
Fig. 9 is a drawing showing the result of assaying the length of inserts of each clone, after generating cDNA library by using an amplified cDNA mixture. Inserts were recognized in all clones with the exception of the lane 12. A marker for DNA molecular weight was electrophoresed in the lane M.

### Best Mode for Carrying out the Invention

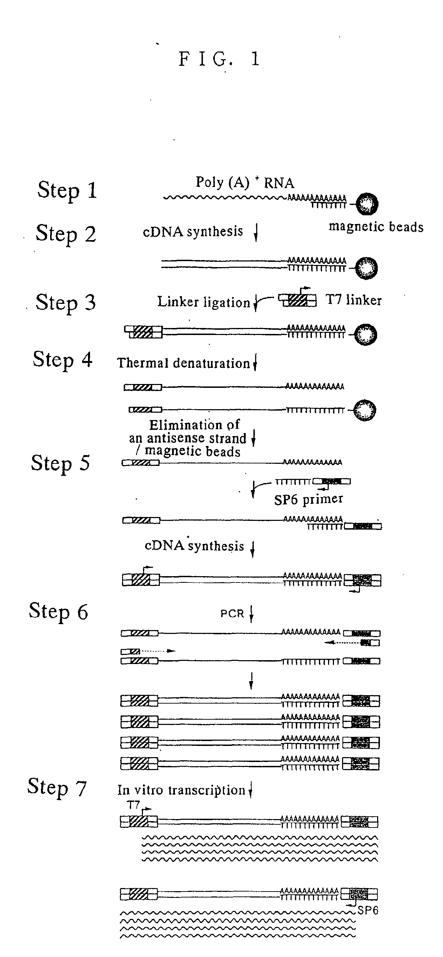

There is no particular limitation to a method for amplifying mRNA in microquantity of the present invention as long as it comprises the following processes, (1) a process of making mRNA in a sample adsorbed to a carrier wherein an oligo (dT) is bound, (2) a process of synthesizing an antisense strand cDNA and a sense strand cDNA on a carrier, (3) a process of adding a linker containing the first promoter sequence of the 5' end of at least sense strand among the double-stranded cDNA obtained herein, (4) a process of dissociating said double-stranded cDNA and eliminating an antisense strand cDNA binding to a carrier together with said carrier, (5) a process of synthesizing a double-stranded cDNA by using said sense strand cDNA dissociated herein as a template and using an oligo (dT) primer wherein a linker containing the second promoter sequence is added, (6) a process of amplifying a cDNA mixture by PCR using a sequence of a linker part of the both ends of a double-stranded cDNA as a primer, or it comprises the following process in addition to the processes (1) to (6) mentioned above, (7) a process of synthesizing a sense strand cRNA and/or an antisense strand cRNA by in vitro transcription system using the first promoter sequence and/or the second promoter sequence of aforementioned. One example of said method is shown in Fig. 1.

There is no particular limitation to a sample used for the process (1) mentioned above as long as it comprises mRNA of a cell or a tissue and the like of an animal, a plant, a microorganism arid the like, and the preparation of a liquid sample containing mRNA of a lysate of these cells and the like can be conducted by ordinary protocols. However, it is preferable to prepare in a buffer solution wherein RNase activity is inhibited in the presence of guanidine thiocyanate and the like. According to the present invention, for example, after dissolving a cell by using a guanidine thioacynate, it is only required to isolate approximately 0.1 ng or more of total RNA contained in approximate one cell, wherein only approximately 5 pg or more of the targeted mRNA which is to be amplified should be contained. Besides, there is no limitation to a carrier used for the process (1) as long as it is water-insoluble, and not melted at heat denaturation. Such carrier can be eligibly exemplified by polyethylene beads, plastic plates, magnetic beads and the like, however, among them, magnetic beads are particularly preferable, with which the operation of eliminating antisense strand cDNA binding to a carrier together with such carrier can be conducted easily. Further, by conducting the processes (1) to (3) on a carrier made of magnetic beads and the like, it will be easy to exchange the reactive liquid, and therefore the loss of a sample will be small.

Any oligo (dT) may be used for the process (1) as long as it is synthesized by ordinary protocols. Polymerization degree of said oligo (dT) is not particularly restricted as long as being possible to hybridize with poly (A) of mRNA and to make mRNA adsorbed to a carrier wherein oligo (dT) is bound, but the extent of 5 - 200, or particularly, 10 - 30 is preferable. Besides, poly U and the like containing a complementary sequence for poly (A) of mRNA can also be used as a substitution of an oligo (dT), and using them is also included in the scope of the present invention. There is no particular limitation to a method for binding said oligo (dT) and a carrier made of magnetic beads and the like as mentioned above, and it is exemplified by a covalent binding method, an ionic binding method, physisorption method, or a method wherein a biotin-avidin system is used and the like.

A reaction in which mRNA in a sample is adsorbed to a carrier wherein an oligo (dT) is bound at the process (1) can be conducted by incubating an oligo (dT) binding carrier and a sample containing poly (A)+ RNA in buffer solution, and hybridizing oligo (dT) binding to a carrier and poly (A) of mRNA. It is preferable to conduct incubation for said hybridization under gentle agitation at 20°C to 25°C for approximately 5 minutes. As for the buffer solution mentioned above, it is preferable to use the buffer solution in which RNase activity is eliminated as much as possible. In addition, it is preferable to wash and eliminate ingredients which are not bound to a carrier in a sample from an insoluble carrier by using buffer and the like mentioned above after incubation.

A carrier-binding oligo (dT)-poly (A)⁺ RNA complex prepared in the aforementioned process (1) is used for synthesis of an antisense strand cDNA and a sense strand cDNA upon a carrier made of magnetic beads and the like in the process (2). Synthesis of an antisense strand cDNA can be conducted by reacting an oligo (dT) as a primer, and mRNA as a template under the presence of deoxynucleotide by using a reverse transcriptase, and preparing a poly (A)⁺ RNA-carrier binding cDNA complex upon a carrier. Synthesis of a sense strand cDNA can be conducted by treating poly (A)⁺ RNA -carrier binding cDNA complex with a liquid containing RNase and digesting and eliminating poly (A)⁺ RNA, or dissociating and eliminating poly (A)⁺ RNA by using dilute NaOH solution, and subsequently or in parallel, reacting DNA polymerase with the use of carrier-binding antisense strand cDNA as a template under the presence of deoxynucleotide, and preparing a sense strand cDNA-carrier-binding antisense strand cDNA complex upon a carrier. However, it is preferable to make DNA ligase present in order to promote the link of fragments of sense strand cDNA. In addition, it is preferable to blunt the 5' end of double-stranded cDNA obtained herein by treating with T4 DNA polymerase.

Subsequently, a linker containing the first promoter sequence is added at the process (3) to the 5' end of at least a sense strand of the carrier-binding double-stranded cDNA obtained at the aforementioned process (2). As for said linker containing the first promoter sequence, any of single strand or double strand can be used as long as it is able to bind to the 5' end of at least a sense strand of carrier-binding double-stranded cDNA by DNA ligase and the like. However, double strand is more preferable in view of operational convenience, and for example, a linker of which the 5' end is a protruding end and the 3' end is a blunt end can be used. As for a linker containing the aforementioned first promoter sequence, it is preferable in most cases to use a linker containing a restriction enzyme recognition site (sequence) on the 5' end and/or the 3' end side of said promoter sequence for the case of analysis of cDNA and the like. However, it is not preferable to use a restriction enzyme corresponding to the aforementioned recognition site before the process (6) wherein cDNA mixture is amplified, since it will possibly digest and degrade cDNA derived from a sample. At the process (3), since the 5' end comprising an oligo (dT) of an antisense strand of carrier-binding double-stranded cDNA is fixed on a carrier made of magnetic beads and the like, such end is masked, therefore, it is possible to certainly bind a linker containing the first promoter sequence to the 5' end of a sense strand cDNA, and it is further designed to be able to specifically link an oligo (dT) primer, wherein a linker containing the second promoter sequence is added, to the 3' end of a sense strand cDNA at the subsequent process (5). Further, if a linker formation containing the aforementioned first promoter sequence is integrated beforehand in a linker which is directly linked to the cap site of mRNA, it will be easy to amplify the full length of cDNA.

As for the first promoter sequence mentioned above, a promoter sequence wherein RNA polymerase being able to specifically transcribe said promoter is preferable. Particularly in the case that the first promoter sequence is different from the second promoter sequence as described hereinafter, for example, if T7 promoter sequence is used as the first promoter sequence and SP6 promoter sequence is used as the second promoter sequence, antisense strand cRNA can be specifically amplified by using T7 polymerase being able to specifically transcribe T7 promoter sequence at the process (7). A promoter sequence which enables the promoter-specific transcription by the aforementioned RNA polymerase can be specifically exemplified by T7 promoter sequence (5'-TAATACGACTCACTATAGGGAGA-3'; SEQ ID NO:6), SP6 promoter sequence (5'-ATTTAGGTGACACTATAGAATAC-3'; SEQ ID NO:7), T3 promoter sequence (5'-AATTAACCCTCACTAAAGGG-3'; SEQ ID NO:8) and the like. And a linker containing these first promoter sequences can be prepared by ordinary protocols by using DNA synthesizer.

As for the aforementioned carrier-binding double-stranded cDNA wherein the linker containing the first promoter sequence on the 5' end is added, at the subsequent process (4), double strand of said cDNA is dissociated and an antisense strand cDNA is eliminated together with a carrier. A method for dissociating a double-stranded cDNA is not particularly restricted, and for example, it is conducted by heat denaturating the double-stranded cDNA by heating in low salt concentration solution at 90-100°C for approximately 1 to 10 minutes. Elimination of antisense strand cDNA-binding carrier after said double-stranded cDNA is dissociated can be conducted by ordinary protocols. For example, it can be eliminated by using a magnetic material such as a magnet if the carrier is made of magnetic beads, and by centrifugation or filtration if the carrier is made of polyethylene beads. However, it is preferable to use magnetic beads as a carrier viewing that the loss of sense strand cDNA which remains free in the solution can be kept to a minimum.

At the subsequent process (5), double-stranded cDNA is synthesized again by using a sense strand cDNA dissociated at the process (4) as a template, and an oligo (dT) primer wherein a linker containing the second promoter sequence is added. After adding an oligo (dT) primer wherein a linker containing the second promoter sequence is added to a solution containing a free sense strand cDNA obtained at the aforementioned process (4), an oligo (dT) of the aforementioned primer is hybridized at a poly (A) part of the 3' end of a sense strand cDNA, and thus a complex of sense strand cDNA and the aforementioned oligo (dT) primer is generated. As for the aforementioned linker containing the second promoter sequence in the oligo (dT) primer wherein a linker containing the second promoter sequence is added, it is preferable to use a linker containing a restriction enzyme recognition sequence on the 5' end and/or the 3' end of said promoter sequence, in most cases for the analysis of cDNA and the like. However, it is not preferable to use a restriction enzyme corresponding to a restriction enzyme recognition site before the process (6) wherein a cDNA mixture is amplified, since such enzyme will possibly digest and degrade cDNA derived from a sample. As for the second promoter sequence mentioned above, a promoter sequence, wherein RNA polymerase is able to start transcription specifically, such as T7 promoter sequence, SP6 promoter sequence, T3 promoter sequence and the like is preferable. Particularly in the case that the second promoter sequence is different from the aforementioned first promoter sequence, for example, if SP6 promoter sequence is used as the second promoter sequence and T7 promoter sequence is used as the first promoter sequence, antisense strand cRNA can be specifically amplified by using SP6 polymerase being able to specifically transcribe SP6 promoter sequence at the process (7). These oligo (dT) primers wherein a linker containing the second promoter sequence is added can be synthesized by ordinary protocols by using a DNA synthesizer.

With the use of a complex of a sense strand cDNA and the aforementioned oligo (dT) primer, and a sense strand cDNA as a template, it is possible to synthesize a double-stranded cDNA wherein a linker part containing the promoter sequence at the both 5' ends is added by reacting DNA polymerase or a reverse transcriptase in the presence of deoxynucleotide. At the process (6), a total cDNA mixture is amplified by conducting PCR using a known sequence of a linker part of the both ends of this double-stranded cDNA as primer. PCR can be conducted by ordinary protocols with the use of thermal cycler (Perkin-Elmer) and the like. Approximately 10 µg of cDNA mixture can be obtained during the processes through (6). It becomes possible to construct a cDNA library by using said cDNA mixture. It is also possible to directly determine the base sequence from cDNA band excised from the gel by diluting a total cDNA mixture into several tens of molecular species, conducting PCR, and separating by electrophoresing such PCR product.

With the use of the aforementioned first promoter sequence and/or second promoter sequence of double-stranded cDNA amplified in large quantities at the process (6) wherein a linker part containing promoter sequences on both ends is added, it is possible to synthesize a large amount of sense strand cRNA and/or antisense strand cRNA by in vitro transcription system using a RNA polymerase. As described above, if the first promoter sequence is different from the second promoter sequence, it is possible to synthesize a sense strand cRNA or antisense strand cRNA separately. At this process (7), approximately 100 µg of a sense strand or antisense strand cRNA mixture can be easily prepared by using approximately 10 µg of cDNA mixture obtained at the processes through (6). This RNA amount of approximately 100 µg is sufficient amount for a conventional molecular biological experiment, and for example, subtraction cloning becomes possible by using a sense strand or antisense strand cRNA mixture.

As mentioned above, by applying a method for amplifying mRNA in microquantity of the present invention, even mRNA in ultramicroquantity which is transiently expressed in vivo can be amplified up to the sufficient amount for a conventional molecular biological experiment. Therefore, a method for amplifying mRNA in microquantity of the present invention can be widely used for detecting a gene, cloning, generating cDNA library, generating and analyzing microarray and the like. A cloning method for a gene of the present invention is not particularly restricted as long as it is a method using the aforementioned method for amplifying mRNA in microquantity of the present invention, and detecting or screening a gene, in addition to cloning of a gene can be conducted by said cloning method for a gene. More specifically, by labeling cDNA or cRNA amplified by a method for amplifying mRNA in microquantity of the present invention, and using these labeled cDNA or cRNA, analysis of reverse-northern hybridization, subtraction cloning, DNA array and the like can be conducted. In addition, it is also possible to conduct conventional southern hybridization or northern hybridization for cDNA or cRNA amplified by the present invention. Further, when the full length of cDNA prepared by an amplification method for mRNA in microquantity of the present invention is used, it is possible to identify the number of genes whose expression level fluctuates, and their expression products by synthesizing proteins by in vitro transcription and translation, and analyzing the proteins by two dimensional electrophoresis and the like.

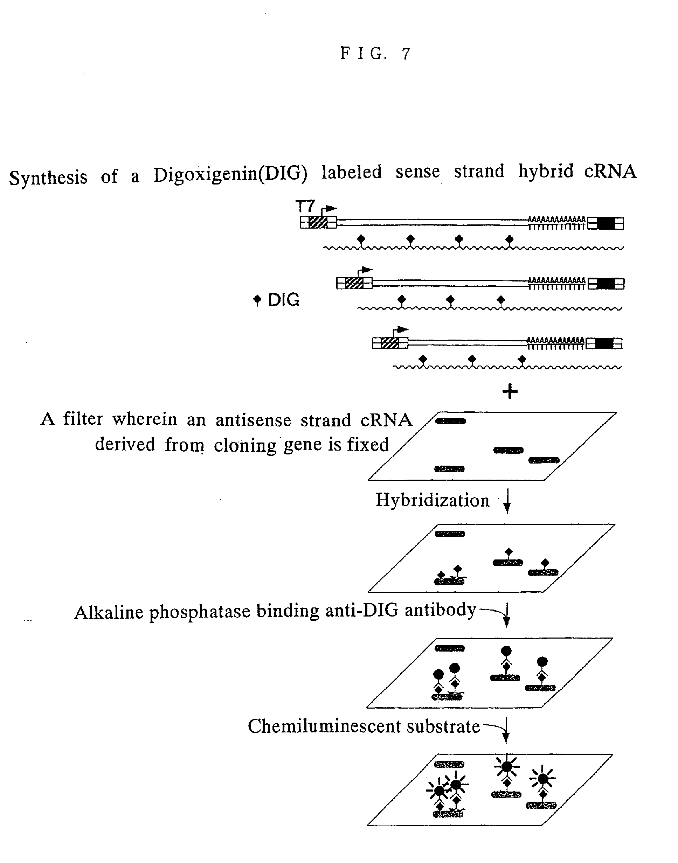

As for a reverse-northern hybridization method mentioned above, as exemplified in Fig. 7, a sense strand cRNA mixture is synthesized in vitro under the presence of substrate ribonucleotide labeled by digoxigenin (DIG) and the like from the amplified double-stranded cDNA mixture obtained by a method for amplifying mRNA in microquantity of the present invention. Reverse northern-hybridization is further conducted by synthesizing an antisense strand cRNA in vitro from the cloned cDNA of a specific gene, electrophoresing said cRNA by using a modified agarose gel, transferring and fixing onto a membrane such as a nylon membrane or nitrocellulose membrane, applying a sense strand cRNA mixture labeled by the aforementioned DIG and the like to said antisense strand cRNA fixed on a membrane, subsequently detecting hybridized cRNA by using, for example, alkaline phosphatase-binding anti-DIG antibody and chemiluminescent substrate. By conducting said reverse-northern hybridization for mRNA derived from a cell and mRNA derived from a control cell under a specific condition separately, and comparing the result of those hybridizations, changes in mRNA level of a gene whose expression level fluctuates in vivo during development or in the presence of a specific agent can be detected.

There is no particular limitation to a subtraction cloning method of the present invention, as long as it is a method for labeling and using at least one among sense strand cDNA, antisense strand cDNA, sense strand cRNA or antisense strand cRNA obtained by the aforementioned method for amplifying mRNA in microquantity of the present invention. Cloning by the following subtraction can be exemplified: a large amount of a sense strand cRNA mixture is prepared as a result of amplifying mRNA derived from cells under a specific condition by a method for amplifying mRNA in microquantity of the present invention. On the other hand, a large amount of antisense cRNA mixture is prepared as a result of amplifying mRNA derived from control cells by a method for amplifying mRNA in microquantity of the present invention. Coupled with the synthesis of said antisense strand cRNA mixture, labeling is performed by using a biotynilated ribonucleotide as a substrate. Subsequently, the aforementioned sense strand cRNA mixture and a biotin-labeled antisense strand cRNA mixture product are hybridized, and reacted with avidin-binding magnetic beads, and then an antisense strand cRNA which is not hybridized, or a complex of a sense strand cRNA and an antisense strand cRNA mixture is eliminated to outside the system by using a magnetic material and the like, a sense strand cRNA derived from mRNA expressing only in cells under a specific condition which does not hybridize is obtained and used as a template in order to synthesize an antisense strand cDNA, cDNA is amplified by PCR, and subsequently, Escherichia coli (E. coli) is transformed by using a plasmid wherein said cDNA is inserted, and thereafter differential hybridization is conducted by ordinary protocols. In said subtraction cloning method, it is possible to start with, for example, an early mouse embryo, or a minute brain nucleus/tissue region of a mouse.

Any microarray can be used as a microarray of the present invention, as long as it is generated by using at least one of a sense strand cDNA, an antisense strand cDNA, a sense strand cRNA or an antisense strand cRNA obtained by the aforementioned method for amplifying mRNA in microquantity of the present invention. Said microarray can be generated by conventionally known methods such as the one described on page 26 to 34 of "DNA Microarray and the Latest PCR Method" (Shujunsha, March 16, 2000) and the like. In addition, genome-wide expression analysis using said microarray can also be conducted by a conventionally known method such as the one described previously (Nature Vol.407, September 7 (2000) Appendix 9-19) and the like.

There is no limitation to a cDNA library of the present invention, as long as a cDNA mixture obtained by a method for amplifying mRNA in microquantity of the present invention is inserted into a vector. Such vectors are exemplified by conventional known vectors for generating a library such as a plasmid vector, a phage vector, a cosmid vector and the like. According to a method for amplifying mRNA in microquantity of the present invention, because it is not necessary to use a restriction enzyme until when an amplified cDNA mixture is prepared by the processes (1) through (6), it does not trigger the deletion of a part of cDNA. Generation of such cDNA library can be started with, for example, an early mouse embryo, or a minute brain nucleus/tissue region of a mouse. In addition, a cDNA mixture which is further synthesized from a cRNA mixture obtained at the process (7) by using a reverse transcriptase can be used as a cDNA mixture for generating cDNA library.

Further, in the prior art (WO93/15228), a part of cDNA is deleted by restriction enzyme digestion, which is conducted to generate a restriction end for insertion of cDNA into a plasmid vector. However, in the present invention, it is possible to insert a single copy of cDNA unidirectionally into a plasmid vector by using a cDNA fragment containing specific restriction end sequences on its both termini, which were generated by using 3' → 5' exonuclease activity of DNA polymerase instead of using restriction enzyme digestion. As explained later in detail with an example, if T4 DNA polymerase works in a reaction mixture containing, for example, dATP and dTTP but not dCTP and dGTP, nucleotide sequences comprising C and/or G is eliminated from the 3' end up to where A or T appears by its 3' → 5' exonuclease activity, and a resultant 5'-protruding end is thus formed, yielding a cDNA fragment containing restriction ends for AvaI and AccI, which enable insertion of a single copy of said cDNA fragment unidirectionally into a plasmid vector.

There is no limitation to an amplification kit for mRNA in microquantity of the present invention, as long as it includes a carrier made of magnetic beads and the like wherein an oligo (dT) is bound, a linker containing the first promoter sequence, an oligo (dT) primer wherein a linker containing the second promoter sequence which is different from said first promoter sequence is added. However, it is preferable to use the one containing various buffer solutions used at the aforementioned processes (1) through (7). With the use of an amplification kit for mRNA in microquantity of the present invention, it is possible to conduct easily the aforementioned subtraction cloning, generation and analysis of microarray, and construction of cDNA library.

The present invention will be further specifically explained in the following with reference to the examples, but the scope of the invention will not be limited to these examples .

### Example 1 [Method for Amplifying mRNA in microquantity (MSMAP); Fig. 1]

### [Preparation of RNA sample solution]

Total RNA was extracted from a primary-cultured rat hepatocytes by Acid Guanidium Thiocyanate-Phenol-Chloroform extraction method (AGPC method), and 10 µl of aqueous solution containing 1 µg of said total RNA was used as a starting material. As a result of serial dilution of said solution with sterile water, 10 µl of each sample solution containing 10², 10, 1, 10⁻¹, 10⁻², 10⁻³ng of RNA respectively and 10 µl of sample solution containing 0 ng of RNA as a negative control were prepared.

### [Adsorption of poly (A)⁺ RNA to oligo (dT) magnetic beads (Step 1, Fig.1)]

10 µl of aqueous solution containing 1 µg of the aforementioned RNA was incubated at 65° C for 5 minutes, quenched on ice, and added to 10 µl of 2 x binding buffer[1 x composition of binding buffer : 10 mM tris hydrochloride (pH 7.5), 0.5 M sodium chloride, 1 mM EDTA] wherein 25 µg of oligo (dT) magnetic beads (Dynabeads Oligo (dT)₂₅, Dynal) had been suspended, and incubated at room temperature for 5 minutes, and then poly (A)⁺ RNA was annealed to an oligo (dT). Poly (A)⁺ RNA-adsorbed oligo (dT) beads were washed for 2 times in 50 µl of 0.3 x binding buffer by repeating aspiration and dispersion by a magnet (MPC-E/E1, Dynal).

### [Synthesis of double-stranded cDNA on magnetic beads (Step 2, Fig.1)]

The aforementioned poly (A)⁺ RNA-adsorbed oligo (dT) magnetic beads are suspended in 20 µl of reaction mixture containing 20 mM tris hydrochloride (pH 8.4), 50 mM potassium chloride, 2.5 mM magnesium chloride, 10 mM DTT, 1 mM dNTP (dATP, dCTP, dGTP, dTTP), 0.1 mg/ml BSA, M-MLV reverse transcriptase (SuperScriptII, Gibco BRL) 200 units, and incubated at 42° C for 50 minutes (with mixing every 10 minutes and suspending beads), and an antisense strand cDNA was thus synthesized. The reaction was stopped by adding 0.5 M EDTA (pH 8.0) 0.8 µl, mRNA/cDNA beads were washed for 3 times in 10 mM tris hydrochloride (pH 8.0)/1 mM EDTA (hereinafter referred to as TE solution) 50 µl. Subsequently, said beads were suspended in 20 µl of reaction mixture containing 19 mM tris hydrochloride (pH 8.3), 91 mM potassium chloride, 4.6 mM magnesium chloride, 10 mM ammonium sulfate, 3.8 mM DTT, 0.15 mM NAD, 1 mM dNTP (dATP, dCTP, dGTP, dTTP), E. coli DNA polymerase I (Gibco BRL) 5 units, E. coli DNA ligase (Gibco BRL) 5 units, E. coli RNaseH (Gibco BRL) 1 unit, and incubated at 16° C for 1 hour, and then a sense strand cDNA was synthesized, yielding magnetic beads-fixed double-stranded cDNA. Further, 1 unit/µl T4 DNA polymerase (Roche Diagnositics) 0.5 µl was added, and incubated at 16° C for 10 minutes, and thus 5' end was thoroughly blunted. The reaction was stopped by adding 0.5 M EDTA (pH 8.0) 0.8 µl, and double-stranded cDNA beads were washed for 3 times in TE solution 50 µl.

### [Addition of a promoter sequence to cDNA 5' end and collection of a sense strand cDNA (Steps 3 and 4, Fig. 1)]

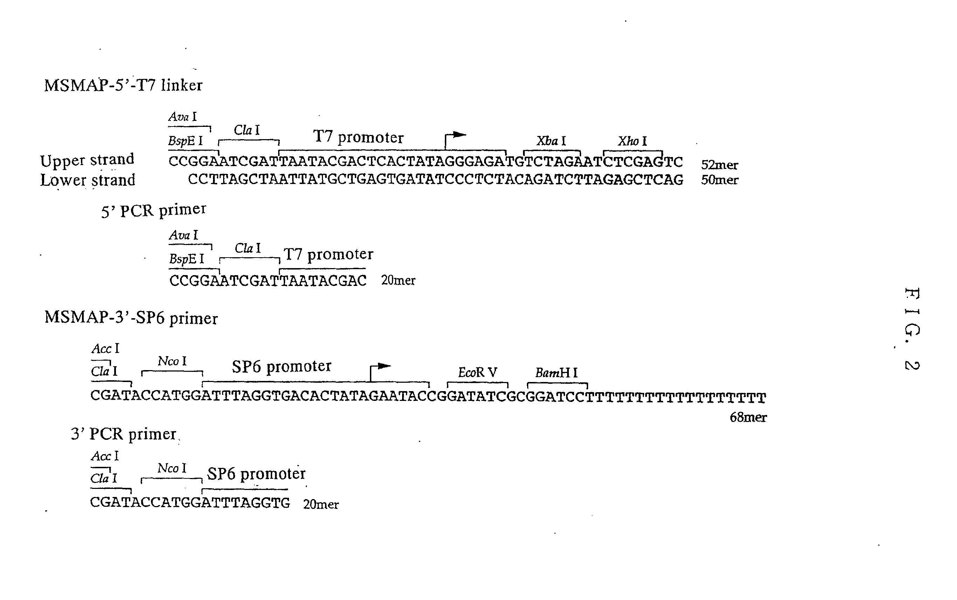

Oligonucleotides of the upper strand comprising 52 mer of the base sequence shown by SEQ ID NO:1 and the lower strand comprising 50 mer of the base sequence shown by SEQ ID NO: 2 were synthesized by ordinary protocols using a DNA synthesizer. The 5' end of the lower strand was phosphorylated by using T4 polynucleotide kinase (Takara Shuzo). The both strands were annealed by ordinary protocols and made to be as a double strand, and as a result, a MSMAP-5'-T7 linker described in Fig. 2 was obtained. The aforementioned double-stranded cDNA beads were suspended in a 20 µl of reaction mixture containing 66 mM tris hydrochloride (pH 7.5), 5 mM magnesium chloride, 5 mM DTT, 1 mM ATP, MSMAP-5'-T7 linker 1 µg, T4 DNA ligase (TAKARA) 350 units (reaction was started finally by adding 1 µl of enzyme solution), and incubated at 4°C for overnight (continually stirred by a rotator), and then, MSMAP-5' -T7 linker was ligated to the 5'end of a double-stranded cDNA (Step 3, Fig. 1). The reaction was stopped by adding 0.5 M EDTA (pH 8.0) 0.8 µl, and a linker-ligated double-stranded cDNA beads were washed for 3 times in TE solution 50 µl. Subsequently, said beads were suspended in TE solution 20 µl, and incubated at 95°C for 5 minutes, dissociating a sense strand cDNA mixture by heat denaturation. Antisense strand cDNA beads were attracted to magnet, and a supernatant containing a sense strand cDNA mixture was collected (Step 4, Fig. 1).

### [Addition of a promoter sequence to the 3' end of cDNA and resynthesis of antisense strand cDNA (Step 5, Fig.1)]

By adding oligo (dT) primer MSMAP-3'-SP6 primer 50 ng, wherein SP6 promoter sequence comprising 68 mer of the base sequence shown by SEQ ID NO:3 is added, to sense strand cDNA solution 4 µl, and as a result total amount of 5 µl was obtained. After being heated at 90° C for 3 minutes and quenched on ice, following components with each final concentration were added: 20 mM tris hydrochloride (pH 8.4); 50 mM potassium chloride; 2.5 mM magnesium chloride; 10 mM DTT; 1 mM dNTP (dATP, dCTP, dGTP, dTTP); 0.1 mg/ml BSA, and preincubated at 42°C for 5 minutes, and M-MLV reverse transcriptase (SuperScriptII, Gibco BRL) 200 units (1 µl) were further added, giving 20 µl of reaction mixture. By incubating the same at 42° C for 1 hour, antisense strand cDNA was synthesized, and a double-stranded cDNA mixture was obtained. After the reaction was finished, it was frozen on dry ice and preserved at -25°C. It can be preserved for at least one year on this condition.

### [Amplification of a cDNA mixture (Step 6, Fig. 1)]

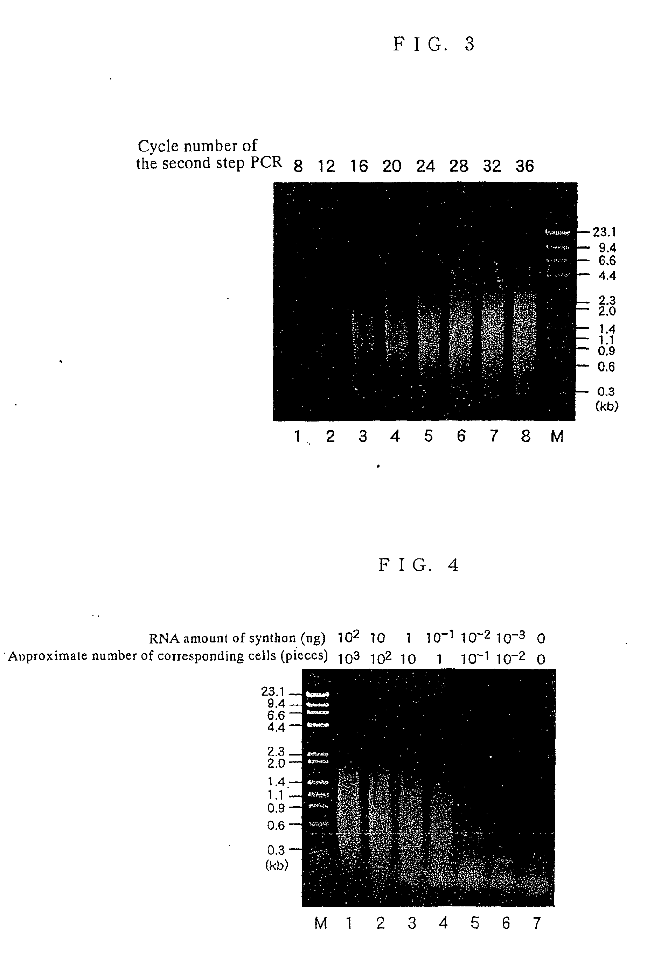

A cDNA mixture was amplified by two-step PCR. Known sequences at the linker parts of both ends of double-stranded cDNA, namely, 5' PCR primer comprising 20 mer of the base sequence shown by SEQ ID NO:4 (Fig.2), and 3' PCR primer comprising 20 mer of the base sequence shown by SEQ ID NO:5 (Fig.2) were used as primers. The first step of PCR was conducted in 100 µl of reaction mixture containing 20 mM tris hydrochloride (pH 8.2) , 10 mM potassium chloride, 6 mM ammonium sulfate, 2 mM magnesium chloride, 0.1% Triton X-100, 0.2 mM dNTP (dATP, dCTP, dGTP, dTTP), 10 µg/ml BSA, the aforementioned double-stranded cDNA solution 2 µl, 5' PCR primer 0.1 nmol, 3'PCR primer 0.1 nmol, heat-stable DNA polymerase (Pfu DNA polymerase, Stratagene) 3 units. PCR condition was as follows; a cycle of heat denaturation at 94° C for 1 minute, followed by annealing at 57°C for 2 minutes and extension at 72°C for 2 minutes was repeated 15 times. At the second step of PCR, 5 µl each of the PCR product mixture obtained as a result of the first step was dispensed into 5 tubes respectively, and the reaction was carried out in 100 µl of mixture in which other components are same as those used at the first step. Besides, PCR condition was same as the first step. 5 tubes of product mixture (corresponding to 1/200 of total RNA 1 µg) were collected into one tube, and reaction was stopped by adding 0.5 M EDTA (pH 8.0) 10 µl and 10% SDS 10 µl. After repeating two times each of the followings : extraction by TE-saturated phenol 500 µl; extraction by TE-saturated phenol/chloroform (50:50) 500 µl; extraction by chloroform 500 µl, 20 µg (1 µl) of glycogen (Roche Diagnostics) were added as a carrier to the approximate 450 µl of remained product mixture, and 2/3 volume of 5M ammonium acetate (300 µl) . and 2 volumes of ethanol (1.5 ml) were further added, kept on ice for 1 hour, and then a product was collected by centrifugation. The pellet was washed in 70% ethanol 1 ml, then air dried, and dissolved into TE solution 20 µl. As a result of the method mentioned above, it was turned out that if the corresponding amount for 1/200 of total RNA 1 µg was applied at the second step of PCR, approximately 10 µg of an amplified cDNA mixture can be generally obtained. As shown in Fig. 3 , when an amplified cDNA mixture at various cycles of the second step of PCR was electrophoresed in 1% agarose gel and stained fluorescently with ethidium bromide, amplification of cDNA with the length of approximately 4000bp was recognized at 12 or more cycles. As aforementioned, approximately 15 cycles wherein the product amount is not saturated were generally applied as a cycle number for the second step of PCR. In addition, as shown in Fig. 4, total RNA of a starting material could be reduced to 0.1 ng. If it is hypothesized that mRNA included in said total RNA is 2 pg, and when the total amount is amplified, 2 mg of amplified cDNA can be theoretically obtained. Therefore it turned out that amplification of 10⁹ times is possible by the end of this step.

### [Synthesis of a cRNA mixture (Step 7, Fig. 1)]

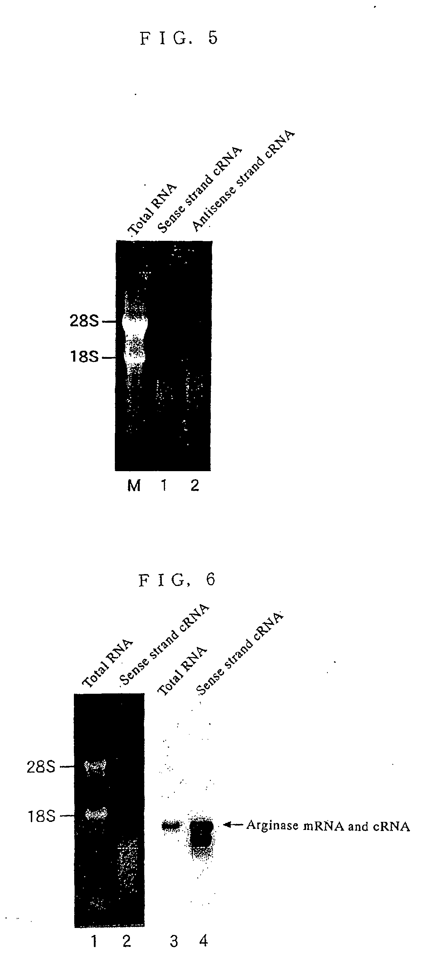

With the use of the aforementioned amplified cDNA mixture as a template, a sense strand and antisense strand cRNA were specifically synthesized by using T7 and SP6 RNA polymerase respectively as described below. 20 µl of a reaction mixture containing the followings: 40 mM tris hydrochloride (pH 8.0); 6 mM magnesium chloride; 10 mM DTT; 2 mM spermidine; 1 mM NTP (ATP, CTP, GTP, UTP); RNase inhibitor (Roche Diagnostics) 4 units; amplified cDNA mixture 0.3 µg; T7 RNA polymerase (Roche Diagnostics) or SP6 RNA polymerase (Roche Diagnostics) 40 units, was incubated at 37° C for 2 hours, and thus cRNA was synthesized. Subsequently, DNase I (10 units/µl, Roche Diagnostics) 2 µl which does not include RNase activity was added, and resulting solution was incubated at 37° C for 15 minutes and thus a template cDNA was digested. Finally, the reaction was stopped by adding 0.5 M EDTA (pH 8.0) 0.8 µl. Subsequently, after 2/3 volume of 5M ammonium acetate (15.2 µl) and 2 volumes of ethanol (76 µl) were added, the resultant solution was kept on ice for 10 minutes, and the product was collected by centrifugation. Said product (pellet) thus collected was washed in 70% ethanol 0.1 ml, air dried, and dissolved into 10 µl of sterile water. Besides, as a result of the aforementioned, it is turned out that approximately 10 µg of an amplified cRNA mixture can be generally obtained from 0.3 µg of amplified cDNA mixture. Based on the aforementioned, it was calculated that 10⁸-fold amplification can be routinely accomplished when started from 1 µg of total RNA (approximately 20 ng mRNA) , and theoretically up to 10¹²-fold amplification when started from 0.1 ng of total RNA. As shown in Fig. 5, when 0.3 µ g of an amplified cRNA mixture was electrophoresed in 1% agarose/MOPS acetate/formaldehyde gel, and stained fluorescently with ethidium bromide, synthesis of cRNA up to approximately 2000 b length was recognized.

### [Northern hybridization analysis of an amplified cRNA mixture (Fig. 6)]

After 2 µg of total RNA derived from a primary-cultured rat hepatocytes, and 0.3 µg of its amplified sense strand cRNA mixture were electrophoresed in 1% agarose/MOPS acetate/formaldehyde gel, they were subjected to RNA fluorescence band detection and further blotted to a nylon membrane by ordinary protocols. Antisense strand cRNA was labeled with DIG by using arginase cDNA as a template and using a kit of Roche Diagnostic product. Hybridization was carried out by using the same as a probe. According to the protocol of said company, a luminescent signal was detected in X-lay film by using alkaline phosphatase-conjugated anti-DIG antibody and chemiluminescent substrate CDP-Star. Approximately 1.6 kb of arginase mRNA and its sense strand cRNA were detected.

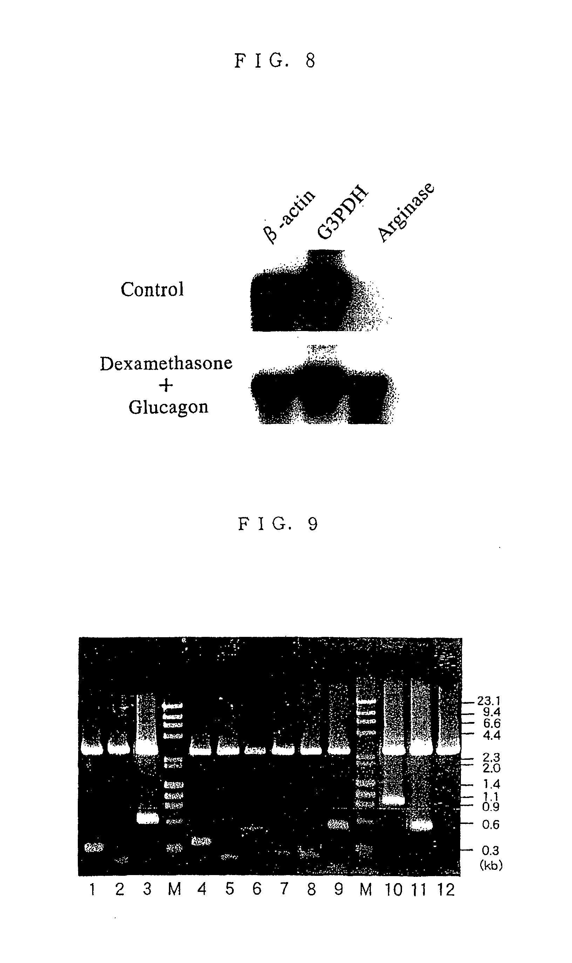

### [Reverse northern hybridization analysis by using a labeled cRNA mixture (Figs. 7, 8)]

The principle and experimental example of the method are shown in Figs.7 and 8, respectively. This method makes it possible to measure the level of specific mRNA as follows: cRNA derived from a cloned gene is fixed on a filter, and a labeled antisense cRNA mixture derived from a sample is hybridized to said filter. An antisense strand cRNA was synthesized by in vitro transcription system using cDNA of β-actin, glyceraldehyde-3-phosphate dehydrogenase (G3PDH), and arginase as templates. After 0.5 µg of each cRNA was electrophoresed in 1% agarose/MOPS acetate/formaldehyde gel, they were blotted onto nylon membrane by ordinary protocols. On the other hand, a DIG-labeled sense strand cRNA mixture was synthesized with the kit produced by Roche Diagnostics, using as a template an amplified cDNA mixture derived from total RNA of a primary-cultured rat hepatocytes which were treated for 2 hours or untreated with 10⁻⁶ M dexamethasone and 3 x 10⁻⁸ M glucagon. According to the protocol of said company, 0.5 µl/ml of a DIG labeled sense strand cRNA mixture was reacted against antisense strand cRNA blot at 68°C for overnight, a signal derived from hybridized RNA was detected onto to X-ray film as chemiluminescence. As a result of the aforementioned, no change was recognized in mRNA level of β-actin and G3PDH, while arginase mRNA level was shown to be elevated in response to dexamethasone and glucagon.

### [Generating cDNA library from cDNA construct (Fig. 9)]

Only a single copy of cDNA construct before or after amplification by PCR can be inserted unidirectionally into a plasmid vector such as pUC18/19, pGEM-3Zf(+)/(-) and the like by using the specific sequence constructed on the both ends of said cDNA mixture. Against the 5' end sequence of cDNA construct mentioned below: 5'-CCGGA······-3' 3'-GGCCT·····-5' when T4 DNA polymerase is made to act in reaction solution containing only dATP and dTTP, but not dCTP and dGTO, the 5'-protruding end of the following: 5'-CCGGA·····-3' 3'-T······-5' can be formed by 3' → 5' exonuclease activity of the enzyme. This end is complementary to the 5'-protruding end which is formed when polylinker sites such as pUC18/19, pGEM-3Zf(+)/(-) and the like are digested with AvaI. In a similar manner, against the 3' end sequence of cDNA construct mentioned: 5'-CGA·····-3' 3'-GCT······-5', 5'-protruding end of the following can be formed:

5'-CGA······-3'

3'-T·····-5'.

This end is complementary to the 5'-protruding end which is formed when polylinker sites such as pUC18/19, pGEM-3Zf(+)/(-) and the like are digested with AccI. By using this characteristic, each cDNA can be inserted unidirectionally into AvaI-AccI sites of plasmid. In addition, because both ends of each cDNA are not phosphorylated, there occurs no ligation between cDNAs, and therefore, only a single copy can be inserted.

An example of the experiment wherein cDNA library was constructed by inserting the aforementioned amplified cDNA mixture into pUC19 is shown below. 100 µl of reaction mixture containing the followings: 50 mM tris hydrochloride (pH 8.8); 7 mM magnesium chloride; 15 mM ammonium sulfate; 0.1 mM EDTA; 10 mM mercaptoethanol; 0.2 mg/ml BSA; 0 .1 mM dATP; 0.1 mM dTTP; amplified cDNA mixture 1.2 µ g; T4 DNA polymerase (Roche Diagnostics) 2.5 units, was incubated at 37° C for 5 minutes, and C and G nucleotide residues were eliminated from the both 3' ends of cDNA by 3' → 5' exonuclease activity. Reaction was stopped by adding 0. 5 M EDTA (pH 8.0) 4.0 µ l, and glycogen (Roche Diagnostics) 20 µ g (1 µl) was added as a carrier. After repeating two times each of the followings: extraction by TE-saturated phenol 100 µ l; extraction by TE-saturated phenol/chloroform (50:50) 100µl; extraction by chloroform 100 µl, 2/3 volume of 5 M ammonium acetate (67 ml), and 2 volumes of ethanol (334 ml) were added to the product mixture, the resultant solution was kept on ice for 10 minutes, and a product was collected by centrifugation. The collected product (pellet) was washed in 70% ethanol 0.5 ml, then air dried, and dissolved into TE solution 20 µl. On the other hand, pUC19 was digested with AvaI and AccI, and electrophoresed in agarose gel. A gel strip containing a band of the vector portion was excised, and then DNA was purified by using Glassmilk (Bio 101). Approximately 5 ng of an AvaI/AccI end-constructed cDNA mixture and approximately 5 ng of pUC19 digested by AvaI/AccI were ligated using T4 DNA ligase, and then E. coli JM109 competent cells were transformed by ordinary protocols. As a result, approximately 200 colonies of transformants were obtained, from which 12 clones were randomly selected, and plasmid was extracted from said 12 clones after liquid culture. This was digested with C1aI and HindIII, and analyzed by electrophoresis in 1% agarose. Its result is shown in Fig. 9. Inserts apparently derived from cDNA were identified in 11 clones, and their lengths were turned out to be approximately 200-1000 bp. Based on the facts mentioned above, it is turned out that by using 1 µg of cDNA mixture, a cDNA library comprising approximately 40,000 clones can be constructed easily by using plasmid as a vector.

### Industrial Applicability

A method for amplifying mRNA in microquantity of the present invention is a method with a versatility wherein mRNA/cDNA in microquantity derived from limited cells or tissues of higher organism such as a human can be amplified, and according to the present invention, by combining cDNA synthesis on magnetic beads, cDNA amplification by PCR, and subsequent in vitro RNA synthesis, amplification of mRNA by approximately 100 million times can be easily accomplished, and it is quite useful for isolation of various cDNA from limited cells since preparation of a library is made possible from a single cell even by amplification of cDNA only. In addition, by using T7 and SP6 promoter sequence ligated to both ends of cDNA, it is possible to specifically synthesize cRNAs of the both sense strand and antisense strand, to perform subtraction cloning, and to prepare various specific labeled probes. Said each strand-specific labeled probe is quite useful for supersensitive analysis such as DNA microarray and the like. It becomes further possible to synthesize protein in vitro by using sense strand cRNA which was specifically synthesized. In addition, it is possible to insert a single copy of cDNA unidirectionally into plasmid by potential restriction enzyme recognition site comprising sequence at each end of cDNA, and this also makes analysis after the cloning easier. Thus, the present invention has high versatility in isolation of cDNA derived from sample in microquantity and in analysis of expression of genes of said cDNA, and is quite effective for finding and exploitation of genetic resources.

## Claims

1. A method for amplifying mRNA in microquantity comprising the following processes (1) to (6):
(1) a process of making mRNA in a sample adsorbed to a carrier wherein an oligo (dT) is bound;
(2) a process of synthesizing an antisense strand cDNA and a sense strand cDNA on a carrier;
(3) a process of adding a linker containing the first promoter sequence of the 5' end of at least sense strand among the double-stranded cDNA obtained herein;
(4) a process of dissociating said double-stranded cDNA and eliminating an antisense strand cDNA binding to a carrier together with said carrier;
(5) a process of synthesizing a double-stranded cDNA by using said sense strand cDNA dissociated herein as a template and using an oligo (dT) primer wherein a linker containing the second promoter sequence is added;
(6) a process of amplifying a cDNA mixture by PCR using a sequence of a linker part of the both ends of a double-stranded cDNA as a primer.

2. A method for amplifying mRNA in microquantity comprising the following processes (1) to (7):
(1) a process of making mRNA in a sample adsorbed to a carrier wherein an oligo (dT) is bound;
(2) a process of synthesizing an antisense strand cDNA and a sense strand cDNA on a carrier;
(3) a process of adding a linker containing the first promoter sequence of the 5' end of at least sense strand among the double-stranded cDNA obtained herein;
(4) a process of dissociating said double-stranded cDNA and eliminating an antisense strand cDNA binding to a carrier together with said carrier;
(5) a process of synthesizing a double-stranded cDNA by using said sense strand cDNA dissociated herein as a template and using an oligo (dT) primer wherein a linker containing the second promoter sequence is added;
(6) a process of amplifying a cDNA mixture by PCR using a sequence of a linker part of the both ends of a double-stranded cDNA as a primer;
(7) a process of synthesizing a sense strand cRNA and/or an antisense strand cRNA by in vitro transcription system using the first promoter sequence and/or the second promoter sequence of aforementioned.

3. A method for amplifying mRNA in microquantity of claim 1 or 2, wherein the carrier is made of magnetic beads.

4. A method for amplifying mRNA in microquantity of any of claims 1 to 3, wherein a linker of which the 5' end is a protruding end and the 3' end is a blunt end as a linker containing the first promoter sequence is used.

5. A method for amplifying mRNA in microquantity of any of claims 1 to 4, wherein a linker containing a restriction enzyme recognition sequence on the 5' end and/or the 3' end of a promoter sequence as a linker containing the first promoter sequence and/or the second promoter sequence is used.

6. A method for amplifying mRNA in microquantity of any of claims 1 to 5, wherein the first promoter sequence is different from the second promoter sequence.

7. A method for amplifying mRNA in microquantity of any of claims 1 to 6, of which promoter is the first promoter and/or the second promoter recognized by a RNA polymerase which can specifically transcribe said promoters.

8. A method for amplifying mRNA in microquantity of claim 7, wherein a RNA polymerase which can transcribe promoter-specifically is selected among that for T7 promoter, SP6 promoter, or T3 promoter.

9. A method for amplifying mRNA in microquantity of any of claims 1 to 8, wherein the linker containing the first promoter sequence comprises the base sequences shown by SEQ ID NO:1 and 2.

10. A method for amplifying mRNA in microquantity of any of claims 1 to 9 wherein an oligo (dT) primer, to which a linker containing the second promoter sequence is added, comprises the base sequence shown by SEQ ID No:3.

11. A method for cloning a gene by using said method for amplifying mRNA in microquantity of any of claims 1 to 10.

12. A method for subtraction cloning labeling and using at least the one among a sense strand cDNA, an antisense strand cDNA, a sense strand cRNA, or an antisense strand cRNA obtained by the method for amplifying mRNA in microquantity of any of claims 1 to 10.

13. A microarray using at least the one among a sense strand cDNA, an antisense strand cDNA, a sense strand cRNA, or an antisense strand cRNA obtained by the method for amplifying mRNA in microquantity of any of claims 1 to 10.

14. A cDNA library wherein a cDNA obtained by the method for amplifying mRNA in microquantity of any of claims 1 to 10 is introduced into a vector.

15. An amplification kit for mRNA in microquantity comprising the followings, a carrier wherein an oligo (dT) is bound, a linker containing the first promoter sequence, and an oligo (dT) primer wherein a linker containing the second promoter sequence different from said first promoter sequence is added.

16. An amplification kit for mRNA in microquantity of claim 15, wherein a carrier is made of magnetic beads.
